(19) Europäisches Patentamt
European Patent Office
Office européen des brevets

(11) Veröffentlichungsnummer : **0 109 622**
**B1**

(12) # EUROPÄISCHE PATENTSCHRIFT

(45) Veröffentlichungstag der Patentschrift : **12.02.86**

(51) Int. Cl.⁴ : **C 07 D267/10**, A 61 K 31/55

(21) Anmeldenummer : **83111275.0**

(22) Anmeldetag : **11.11.83**

(54) **7-Phenyl-7-phenoxymethyl-hexahydro-1,4-oxazepine, ihre Herstellung und Verwendung.**

(30) Priorität : **20.11.82 DE 3242923**

(43) Veröffentlichungstag der Anmeldung : **30.05.84 Patentblatt 84/22**

(45) Bekanntmachung des Hinweises auf die Patenterteilung : **12.02.86 Patentblatt 86/07**

(84) Benannte Vertragsstaaten : **AT BE CH DE FR GB IT LI NL SE**

(56) Entgegenhaltungen :
**DE-A- 2 901 180**
**GB-A- 2 056 439**
**US-A- 3 391 149**

(73) Patentinhaber : **BASF Aktiengesellschaft**
**Carl-Bosch-Strasse 38**
**D-6700 Ludwigshafen (DE)**

(72) Erfinder : **Treiber, Hans Joerg, Dr.**
**Sperberweg 1**
**D-6831 Bruehl (DE)**
Erfinder : **Hofmann, Hans Peter, Dr.**
**Untere Hart 12**
**D-6703 Limburgerhof (DE)**
Erfinder : **Kreiskott, Horst, Prof. Dr.**
**Am Boehlig**
**D-6706 Wachenheim (DE)**
Erfinder : **Teschendorf, Hans-Juergen, Dr.**
**Georg-Nuss-Strasse 5**
**D-6724 Dudenhofen (DE)**
Erfinder : **Traut, Martin, Dr.**
**Muehltalstrasse 125**
**D-6900 Heidelberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 109 622 B1

**Beschreibung**

Die Erfindung betrifft neue 7-Phenyl-7-phenoxymethyl-hexahydro-1,4-oxazepine, Verfahren zu deren Herstellung sowie deren Verwendung bei der Bekämpfung von psychischen Krankheiten.

Aus der DE-OS-2 901 180 sind bereits 7,7-substituierte Oxazepine bekannt, die eine analgetische Wirkung besitzen.

Es wurden nun strukturell veränderte Verbindungen mit einem anderen Wirkungsspektrum gefunden.

Gegenstand der Erfindung sind 7-Phenyl-7-phenoxymethyl-hexahydro-1,4-oxazepine der Formel I

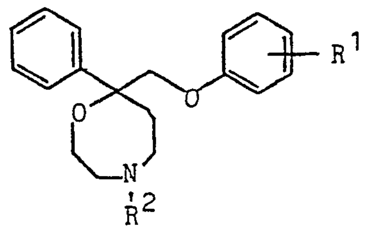

(I)

worin

$R^1$ ein Wasserstoff- oder Chloratom oder eine Methoxygruppe und

$R^2$ ein Wasserstoffatom oder eine Methylgruppe bedeuten,

sowie deren Salze mit physiologisch verträglichen Säuren.

Die Verbindungen der Formel I lassen sich herstellen, indem man

a) — falls $R^2$ eine Methylgruppe bedeutet — eine Verbindung der Formel II

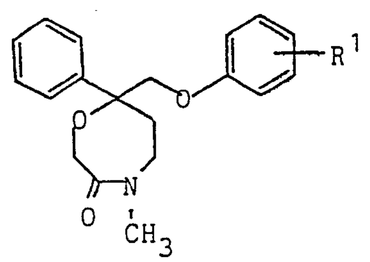

(II)

worin $R^1$ die angegebene Bedeutung hat, mit einem starken Reduktionsmittel reduziert oder

b) — falls $R^2$ ein Wasserstoffatom bedeutet — eine Verbindung der Formel III

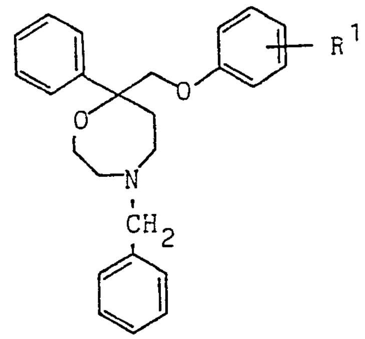

(III)

worin $R^1$ die angegebene Bedeutung hat,
reduktiv debenzyliert und die so erhaltenen Verbindungen gegebenenfalls am Stickstoffatom methyliert und/oder und ihre Salze mit physiologisch verträglichen Säuren übergeführt.

Zur Reduktion der Verbindungen II benötigt man starke Reduktionsmittel, wie Diboran oder vorzugsweise Lithiumaluminiumhydrid, wobei als Lösungsmittel besonders Tetrahydrofuran, Dioxan oder Ether geeignet sind. Die Reduktion wird bei erhöhter Temperatur, vorzugsweise bei Siedetemperatur, des Lösungsmittels durchgeführt.

Die Methylierung von Verbindungen der Formel I, worin R ein Wasserstoffatom bedeutet, kann in herkömmlicher Weise erfolgen. Am einfachsten ist die Methode nach Leuckart-Wallach mit Formaldehyd/Ameisensäure.

Die reduktive Debenzylierung der Verbindungen III wird in bekannter Weise mit Edelmetallkatalysatoren in einem geeigneten Lösungsmittel bei Temperaturen zwischen 0 und 100 °C durchgeführt. Als Katalysator wird vorzugsweise Palladium auf Kohle verwendet. Als Lösungsmittel haben sich niedere Alkohole, wie Methanol oder Ethanol, sowie Essigsäure bewährt.

Die Verbindungen enthalten ein asymmetrisches Kohlenstoffatom, so daß die Verbindungen auch in Form ihrer optischen Antipoden durch eine Racematspaltung erhalten werden können.

Die zur Herstellung der neuen Verbindungen benötigten Ausgangsstoffe der allgemeinen Formeln II und III sind noch nicht beschrieben. Sie lassen sich wie folgt darstellen :

Durch Addition von metalliertem Acetonitril an der Phenoxygruppe durch $R^1$ substituierte ω-Phenoxyacetophenone erhält man in bekannter Weise die Hydroxynitrile der Formel IV

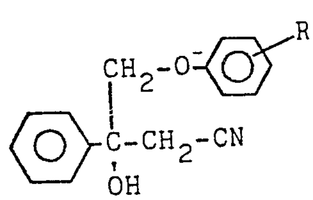

(IV)

Diese lassen sich katalytisch unter Druck mit Raney-Nickel in Methanol zu den entsprechenden Aminen reduzieren. Letztere werden am Stickstoff methyliert bzw. benzyliert und anschließend mit Chloracetylchlorid in Gegenwart von verdünnter Natronlauge in die Verbindungen der Formel V übergeführt

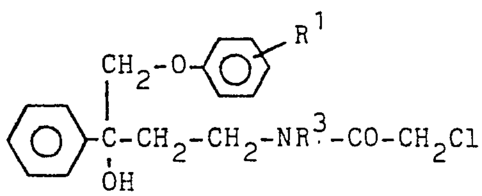

(V)

worin $R^3$ eine Methyl- oder Benzylgruppe darstellt.

Aus den Verbindungen V erhält man durch Erwärmen mit Natrium-isopropanolat in Hexan die Verbindungen der Formel II bzw. die entsprechenden N-Benzylverbindungen. Letztere können durch Hydrierung mit Diboran in die Verbindungen III übergeführt werden.

Die erfindungsgemäßen Verbindungen zeichnen sich durch antidepressive Wirkqualitäten aus. Sie eignen sich daher zur Pharmakotherapie von psychischen Erkrankungen, besonders von Depressionen.

Als Wirkungsmechanismus einer Gruppe therapeutisch vielfach genutzter Antidepressiva ist eine Hemmung der neuronalen Aufnahme von Transmittersubstanzen (Noradrenalin und/oder Serotonin) anzusehen. Diese Eigenschaft wurde in biologischen Testmodellen zur Charakterisierung von potentiellen Antidepressiva verwendet (Hemmung der Neurotransmitter-Aufnahme in Rattenhirn-Synaptosomen).

Hippocampus und Cortex aus Rattenhirn wurden präpariert und in 0,32 M Sucrose-Lösung homogenisiert. Durch differentielle Zentrifugation wurden Synaptosomen erhalten, die in Pufferlösung suspendiert wurden. Die Synaptosomen sind in der Lage, aus dem umgebenden Medium zugefügte Neurotransmittersubstanzen (z. B. Noradrenalin oder Serotonin) aktiv aufzunehmen. Dieser Vorgang ist durch uptake-Hemmer konzentrationsabhängig antagonisierbar. Die Synaptosomen wurden mit den Testsubstanzen in verschiedenen Konzentrationen versetzt und anschließend mit $^3$H-Noradrenalin (Hippocampus) bzw. $^3$H-Serotonin (Cortex) bei 37 °C inkubiert. Die Substratkonzentration betrug ca. 10 nM. Nach Stoppen der Aufnahme durch Verdünnen mit eisgekühlter Pufferlösung wurden die Synaptosomen abzentrifugiert und die $^3$H-Aktivität im Sediment gemessen. Der Blindwert wird durch Inkubation bei 0 °C ermittelt.

Aus den bei verschiedenen Inhibitor-Konzentrationen gegen Kontrolle ermittelten Hemmwerten wurde durch lineare Regression nach logit-log-Transformation die mittlere Hemmkonzentration ($IC_{50}$) berechnet.

In diesem Testmodell (siehe Tabelle 1) zeichnet sich das Standard-Antidepressivum Imipramin dadurch aus, daß es sowohl die Noradrenalin-Aufnahme, als auch die Serotonin-Aufnahme in niedrigem Konzentrationsbereich hemmt, wobei die Hemmung der Noradrenalin-Aufnahme deutlich im Vordergrund steht. Die neuen Substanzen erreichen bzw. übertreffen die Wirkung des Imipramin (Tabelle 1).

In der Noradrenalin-Aufnahme-Hemmung sind die neuen Substanzen bis zu 5fach (Beispiel 2), in der Serotonin-Aufnahme-Hemmung bis zu 3fach (Beispiel 4) stärker wirksam.

Während durch Imipramin die Noradrenalin-Aufnahme 9,3fach stärker gehemmt wird als die Serotonin-Aufnahme, zeichnen sich einige der neuen Substanzen (Beispiel 1, 2, 3) durch eine noch selektivere Hemmung der Noradrenalin-Aufnahme aus, wie sich aus dem im Vergleich zu Imipramin

höheren Quotienten in Tabelle 1 ergibt. Diese Substanzen erscheinen geeignet zur Behandlung von solchen Depressionen, die durch eine Störung des Noradrenalin-Stoffwechsels bedingt sind.

Andererseits zeichnen sich einige der neuen Substanzen (Beispiel 4, 5, 7) durch eine Hemmung der Aufnahme beider Transmitter in gleich niedrigem Konzentrationsbereich aus (Quotient deutlich kleiner als bei Imipramin). Dies deutet auf ein breites Wirkungsspektrum bei der therapeutischen Anwendung hin, da sowohl Depressionen beeinflußt werden könnten, die auf einem gestörten Noradrenalin-Stoffwechsel beruhen, als auch solche, die durch eine Störung des Serotonin-Metabolismus bedingt sind.

Die gefundenen antidepressiven Wirkungen sind insofern überraschend, als bei chemisch nah verwandten Verbindungen (DE-OS-2 901 108) ausgeprägte analgetische, aber keine antidepressiven Eigenschaften gefunden wurden. Die neuen Verbindungen besitzen dagegen keine analgetischen Wirkungen.

Tabelle 1

| Substanz des Beispiels Nr. | Hemmung der Neurotransmitter-Aufnahme in Synaptosomen | | |
|---|---|---|---|
| | Noradrenalin $IC_{50}$ (µmol/l) | Serotonin $IC_{50}$ (µmol/l) | Quotient $\frac{IC_{50}\ \text{Serotonin}}{IC_{50}\ \text{Noradrenalin}}$ |
| 1 | 0,015 | 0,19 | 12,7 |
| 2 | 0,0028 | 0,46 | 164,0 |
| 3 | 0,0059 | 0,19 | 32,0 |
| 4 | 0,074 | 0,039 | 0,53 |
| 5 | 0,086 | 0,10 | 1,2 |
| 7 | 0,098 | 0,30 | 3,1 |
| Imipramin | 0,014 | 0,13 | 9,3 |

Gegenstand der Erfindung sind daher auch Arzneimittel, die eine Verbindung der Formel I enthalten, sowie deren Verwendung bei der Bekämpfung von psychischen Krankheiten.

Die erfindungsgemäßen Verbindungen können in üblicher Weise oral oder parenteral verabfolgt werden.

Die Dosierung hängt vom Alter, Zustand und Gewicht des Patienten sowie von der Applikationsart ab. In der Regel beträgt die tägliche Wirkstoffdosis zwischen etwa 0,1 und 10 mg/kg Körpergewicht.

Die neuen Verbindungen können in den gebräuchlichen galenischen Applikationsformen fest oder flüssig angewendet werden, z. B. als Tabletten, Film-tabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al : Pharmazeutische Technologie, Thieme-Verlag, Stuttgart, 1978). Die so erhaltenen Applikationsformen enthalten den Wirkstoff normalerweise in einer Menge von 1 bis 99 Gew.%.

Die neuen Substanzen können auch in Form ihrer Salze mit physiologisch verträglichen Säuren verabfolgt werden. Solche Säuren sind beispielsweise Salzsäure, Schwefelsäure, Phosphorsäure, Weinsäure, Essigsäure, Citronensäure, Bernsteinsäure, Milchsäure und Amidosulfonsäure.

Die folgenden Beispiele erläutern die Erfindung.

I. Herstellung der Ausgangsstoffe :

a) Zu 50 ml einer auf – 70 °C gekühlten 15 %igen Lösung von n-Butyllithium in Hexan gab man unter Stickstoff 50 ml absolutes Tetrahydrofuran und tropfte dann bei dieser Temperatur 2,8 g (0,07 Mol) Acetonitril in 75 ml Tetrahydrofuran unter Rühren zu. Nach 1 h fügte man 15 g (0,07 Mol) ω-Phenoxyacetophenon, gelöst in 75 ml Tetrahydrofuran innerhalb 5 min zu, entfernte das Kältebad und ließ den Ansatz auf Raumtemperatur kommen.

Die Reaktionsmischung wurde in 1 l Eiswasser und 35 ml 2-N-Salzsäure gegeben und 3x mit je 100 ml Ether extrahiert. Der nach Abdestillieren des Ethers hinterbleibende Rückstand wurde im Vakuum destilliert. Man erhielt 15 g (86 %) 3-Hydroxy-3-phenyl-4-phenoxybutyronitril, $Kp_{0,07\ bar}$ = 175 bis 185 °C.

Ebenso wurden erhalten :

3-Hydroxy-3-phenyl-4-(2-chlorphenoxyl)-butyronitril

3-Hydroxy-3-phenyl-4-(2-methoxyphenoxy)-butyronitril
3-Hydroxy-3-phenyl-4-(4-chlorphenoxy)-butyronitril
3-Hydroxy-3-phenyl-4-(4-methoxyphenoxy)-butyronitril

b) 50,6 g (0,2 Mol) des gemäß a) erhaltenen 3-Hydroxy-3-phenyl-4-phenoxybutyronitrils wurden in 250 ml Methanol gelöst und mit 10 g Raney-Nickel Katalysator 4 h im Autoklav bei 100 °C und 100 bar Wasserstoffdruck hydriert.

Nach Abfiltrieren des Katalysators wurde die Lösung eingedampft und der Rückstand unter vermindertem Druck destilliert. Man erhielt 35,5 g (68 %) 3-Hydroxy-3-phenyl-4-phenoxybutylamin, $Kp_{0,01\ bar}$ = 190 bis 195 °C, Fp = 94 °C.

Ebenso wurden erhalten :
3-Hydroxy-3-phenyl-4-(2-chlorphenoxy)-butylamin
3-Hydroxy-3-phenyl-4-(2-methoxyphenoxy)-butylamin
3-Hydroxy-3-phenyl-4-(4-chlorphenoxy)-butylamin
3-Hydroxy-3-phenyl-4-(4-methoxyphenoxy)-butylamin

c) Eine Lösung von 35 g (0,32 Mol) des gemäß b) erhaltenen 3-Hydroxy-3-phenyl-4-(4-chlorphenoxy)-butylamins in 700 ml Toluol wurde mit 34 g Benzaldehyd versetzt und bis zur Entfernung des Wassers am Wasserabscheider gekocht. Die Lösung wurde eingedampft und der Rückstand in 500 ml Methanol gelöst und nach und nach mit 25 g Natriumborhydrid versetzt. Die Reaktionsmischung wurde eingedampft, Rückstand mit 2N-Natronlauge versetzt und 3x mit je 200 ml Ether extrahiert. Nach Entfernen des Ethers hinterblieb ein Rückstand der durch Umlösen aus Isopropanol kristallin erhalten wurde. Man erhielt 83 g (68 %) N-Benzyl-3-hydroxy-3-phenyl-4-(4-chlorphenoxy)-butylamin, Fp. 84 bis 85 °C.

Analog wurden erhalten :
— N-Benzyl-3-hydroxy-3-phenyl-4-phenoxybutylamin, Fp. 70 bis 71 °C,
— N-Benzyl-3-hydroxy-3-phenyl-4-(2-methoxyphenoxy)-butylamin, Fp. 145 bis 150 °C (Oxalat),
— N-Benzyl-3-hydroxy-3-phenyl-4(2-chlorphenoxy)-butylamin,
— N-Benzyl-3-hydroxy-3-phenyl-4(4-methoxyphenoxy)-butylamin, Fp. 214 bis 215 °C (Oxalat)

d) 45 g (0,13 Mol) gemäß c) hergestelltes N-Benzyl-3-hydroxy-3-phenyl-4-phenoxybutylamin wurden in 400 ml Ethanol gelöst und nacheinander in der Siedehitze mit 7,8 g Ameisensäure und 20 g Formaldehydlösung (35 %) versetzt. Nach 3 h wurde der Alkohol abdestiliert, der Rückstand mit 200 ml 2N-Natronlauge versetzt und 3x mit je 2 ml Ether extrahiert. Nach dem Trocknen der Lösung wurde eingedampft, der Rückstand in 250 ml Essigsäure aufgenommen und in der Schüttelente mit 5 g Palladium auf Kohle Katalysator hydriert. Nach Beendigung der Wasserstoffaufnahme wurde filtriert, eingedampft, mit 2N-Natronlauge versetzt und 3x mit je 200 ml Ether extrahiert. Nach Abdestillieren des Lösungsmittels erstarrte das Produkt. Man erhielt 22 g (65 %) N-Methyl-3-hydroxy-3-phenyl-4-phenoxybutylamin, Fp. 98 bis 99 °C.

e) 60 g (0,17 Mol) des nach c) erhaltenen N-Benzyl-3-hydroxy-3-phenyl-4-phenoxybutylamins wurden in 600 ml Ether gelöst, 150 ml 2N-Natronlauge zugefügt und bei Raumtemperatur 22,6 g (0,2 Mol) Chloracetylchlorid in 50 ml Ether zugetropft. Nach 30 min Kochen unter Rückfluß wurde die etherische Phase abgetrennt, getrocknet und mit 150 ml Isopropanol versetzt. In diese Lösung tropfte man dann in 30 min eine Lösung von 3,4 g Natrium in 375 ml Isopropanol, die mit 1 200 ml Hexan verdünnt war, ein. Nach 2 h Rühren ließ man über Nacht stehen, destillierte das Lösungsmittel ab, versetzte mit Wasser und extrahierte das Produkt mit Chloroform. Nach Trocknen und Eindampfen hinterblieb 4-Benzyl-3-oxo-7-phenyl-7-phenoxymethyl-hexahydro-1,4-oxazepin, das roh weiterverarbeitet wurde.

Ebenso wurden hergestellt :
— 4-Benzyl-3-oxo-7-phenyl-7-(2-chlorphenoxy)-hexahydro-1,4-oxazepin
— 4-Benzyl-3-oxo-7-phenyl-7-(2-methoxyphenoxy)-hexahydro-1,4-oxazepin
— 4-Benzyl-3-oxo-7-phenyl-7-(4-chlorphenoxy)-hexahydro-1,4-oxazepin
— 4-Benzyl-3-oxo-7-phenyl-7-(4-methoxyphenoxy)-hexahydro-1,4-oxazepin
— 4-Benzyl-3-oxo-7-phenyl-7-phenoxy-hexahydro-1,4-oxazepin

f) 21 g (0,05 Mol) des nach e) erhaltenen 4-Benzyl-3-oxo-7-phenyl-7-(2-chlorphenoxy)-hexahydro-1,4-oxazepins wurden mit 5 g Lithiumaluminiumhydrid in 400 ml Tetrahydofuran mehrere h unter Rühren zum Sieden erhitzt. Nach Aufarbeitung mit Wasser und Natronlauge und Eindampfen der Lösungsmittelphase wurde rohes 4-Benzyl-7-phenyl-7-(2-chlorphenoxymethyl)-hexahydro-1,4-oxazepin als zähes Öl erhalten. Es wurde durch Umkristallisieren ihres Oxalates gereinigt. Ausbeute : 14 g (69 %), Fp. 114 bis 115 °C (Oxalat).

Ebenso wurden erhalten :
— 4-Benzyl-7-phenyl-7-(2-methoxyphenoxymethyl)-hexahydro-1,4-oxazepin
— 4-Benzyl-7-phenyl-7-(4-chlorphenoxymethyl)-hexahydro-1,4-oxazepin
— 4-Benzyl-7-phenyl-7-(4-methoxyphenoxymethyl)-hexahydro-1,4-oxazepin-Hydrochlorid, Fp. 179 bis 180 °C.

Herstellung des Endprodukts

Beispiel 1

45 g (0,12 Mol) des gemäß f) erhaltenen 4-Benzyl-7-phenyl-7-phenoxymethyl-hexahydro-1,4-oxazepin-hydrochlorids wurden in 500 ml Essigsäure gelöst und mit 10 g 5 %igem Palladium-Kohle-Katalysator in der Schüttelente unter Normaldruck bei Raumtemperatur hydriert. Nach Aufnahme der berechneten Menge Wasserstoff wurde der Katalysator entfernt, die Essigsäure abdestilliert und der Rückstand aus Essigsäureethylester oder Isopropanol umkristallisiert. Man erhielt 29,5 g (77 %) 7-Phenyl-7-phenoxymethyl-hexahydro-1,4-oxazepin-hydrochlorid, Fp. 95 bis 96 °C.

Ebenso wurden erhalten :

2. 7-Phenyl-7-(2-chlorphenoxymethyl)-hexahydro-1,4-oxazepin-hydrochlorid, Fp. 175 bis 176 °C,
3. 7-Phenyl-7-(2-methoxyphenoxymethyl)-hexahydro-1,4-oxazepin-hydrochlorid, Fp. 183 bis 184 °C,
4. 7-Phenyl-7-(4-chlorphenoxymethyl)-hexahydro-1,4-oxazepin, $Kp_{0,07\ mbar}$ = 190 °C,
5. 7-Phenyl-7-(4-methoxyphenoxymethyl)-hexahydro-1,4-oxazepin-hydrochlorid, Fp. 109 bis 110 °C.

Beispiel 6

7 g (0,022 Mol) des gemäß e) erhaltenen 4-Methyl-3-oxo-7-phenyl-7-phenoxy-methyl-hexahydro-oxazepins wurden in 50 ml Tetrahydrofuran gelöst und langsam in eine siedende Lösung von 3 g Lithiumaluminiumhydrid in 100 ml Tetrahydrofuran getropft. Es wurde 6 h unter Rühren zum Sieden erhitzt, dann in üblicher Weise mit Wasser und 2N-Natronlauge zersetzt und die rohe Base mit etherischer Salzsäure versetzt. Das zunächst ölige Hydrochlorid wurde mit Isopropanol kristallin erhalten. Ausbeute : 4,9 g (65 %) 4-Methyl-7-phenyl-7-phenoxymethyl-hexahydro-1,4-oxazepin. Fp. 218 °C (Hydrochlorid).

Beispiel 7

9,1 g (0,028 Mol) 7-Phenyl-7-(2-chlorphenoxymethyl)-hexahydro-1,4-oxazepin (vgl. Beispiel 6) wurden in 150 ml Ethanol gelöst und in der Siedehitze mit 1,1 g Ameisensäure versetzt. Nach 15 min tropfte man 3,9 g einer 35 %igen Formaldehydlösung langsam zu und hielt die Reaktion noch 2 h am Sieden. Anschließend wurde das Lösungsmittel abdestilliert, der Rückstand mit 50 ml 2N-Natronlauge versetzt und 3 mal mit je 100 ml Ether extrahiert. Aus der getrockneten etherischen Lösung wurde durch Einleiten von Salzsäuregas das zunächst ölige Hydrochlorid erhalten. Es kristallisierte aus 4 Teilen Isopropanol. Ausbeute : 7,6 g (78 %) 4-Methyl-7-phenyl-7-(2-chlorphenoxymethyl)-hexahydro-1,3-oxazepin, Fp. 197 bis 198 °C (Hydrochlorid).

Galenische Beispiele

Beispiel A

Auf einer Tablettenpresse wurden in üblicher Weise Tabletten folgender Zusammensetzung gepreßt :

10,00 mg 7-Phenyl-7-phenoxymethyl-hexahydro-1,3-oxazepin-hydrochlorid
50,00 mg Maisstärke
4,50 mg Gelatine
15,00 mg Milchzucker
7,50 mg Talk
0,75 mg Aerosil® (chemisch reine Kieselsäure in submikroskopisch feiner Verteilung)
2,25 mg Kartoffelstärke (als 6 %iger Kleister)

Beispiel B

In üblicher Weise wurden Dragees folgender Zusammensetzung hergestellt :

10,00 mg 7-Phenyl-7-phenoxymethyl-hexahydro-1,4-oxazepin-hydrochlorid
50,00 mg Kernmasse
40,00 mg Verzuckerungsmasse

Die Kernmasse bestand aus 9 Teilen Maisstärke, 3 Teilen Milchzucker und 1 Teil Luviskol® VA 64 (Vinylpyrrolidon-Vinylacetat-Mischpolymerisat 60 : 40, vgl. Pharm. Ind. *1962*, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel C

Es wurden 5,0 g 7-Phenyl-7-phenoxymethyl-hexahydro-1,4-oxazepin-hydrochlorid in 2,0 l Wasser gelöst, mit Kochsalz isotonisch eingestellt und in 2 ml fassende Ampullen steril abgefüllt.

**Patentansprüche** (für die Vertragsstaaten : BE, CH, DE, FR, GB, IT, LI, NL, SE)

1. 7-Phenyl-7-phenoxymethyl-hexahydro-1,4-oxazepine der Formel I

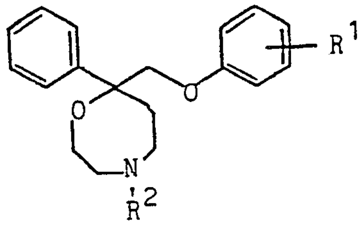

(I)

worin
$R^1$ ein Wasserstoff- oder Chloratom oder eine Methoxygruppe und
$R^2$ ein Wasserstoffatom oder eine Methylgruppe bedeuten,
sowie deren Salze mit physiologisch verträglichen Säuren.

2. 7-Phenyl-7-phenoxymethyl-hexahydro-1,4-oxazepin.

3. Verfahren zur Herstellung der Verbindungen der Formel I gemäß Anspruch 1, dadurch gekennzeichnet, daß man

a) — falls $R^2$ eine Methylgruppe bedeutet — eine Verbindung der Formel II

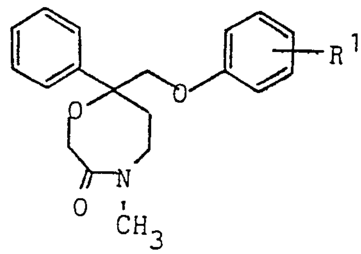

(II)

worin $R^1$ die angegebene Bedeutung hat, mit einem starken Reduktionsmittel reduziert oder

b) — falls $R^2$ ein Wasserstoffatom bedeutet — eine Verbindung der Formel III

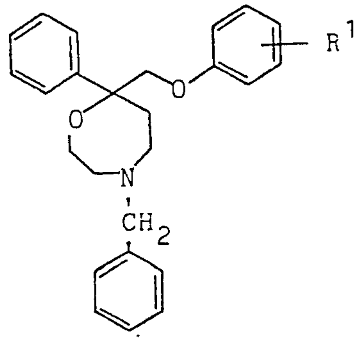

(III)

worin $R^1$ die angegebene bedeutung hat,
reduktiv debenzyliert und die so erhaltenen Verbindungen gegebenenfalls am Stickstoffatom methyliert und/oder und ihre Salze mit physiologisch verträglichen Säuren überführt.

4. Arzneimittel, enthaltend eine Verbindung gemäß Anspruch 1.

5. Verbindung gemäß Anspruch 1 zur Verwendung bei der Bekämpfung von psychischen Krankheiten.

**Patentansprüche** (für den Vertragsstaat AT)

1. Verfahren zur Herstellung von 7-Phenyl-7-phenoxymethyl-hexahydro-1,4-oxazepinen der Formel I

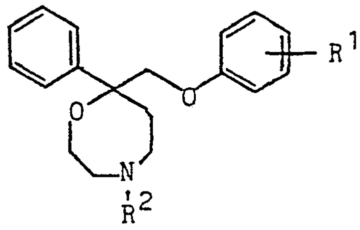

(I)

worin

$R^1$ ein Wasserstoff- oder Chloratom oder eine Methoxygruppe und

$R^2$ ein Wasserstoffatom oder eine Methylgruppe bedeuten,

sowie deren Salze mit physiologisch verträglichen Säuren, dadurch gekennzeichnet, daß man

a) — falls $R^2$ eine Methylgruppe bedeutet — eine Verbindung der Formel II

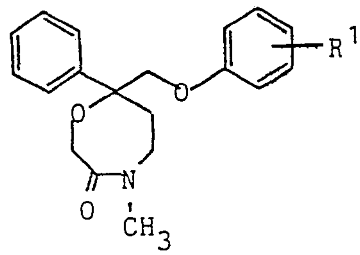

(II)

worin $R^1$ die angegebene Bedeutung hat, mit einem starken Reduktionsmittel reduziert oder

b) — falls $R^2$ ein Wasserstoffatom bedeutet — eine Verbindung der Formel III

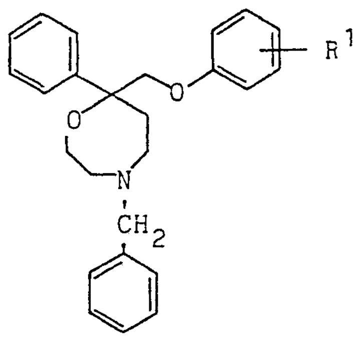

(III)

worin $R^1$ die angegebene Bedeutung hat, reduktiv debenzyliert und die so erhaltenen Verbindungen gegebenenfalls am Stickstoffatom methyliert und/oder und ihre Salze mit physiologisch verträglichen Säuren überführt.

2. Verfahren gemäß Anspruch 1, dadurch gekennzeichnet, daß man 7-Phenyl-7-phenoxymethyl-hexahydro-1,4-oxazepin herstellt.

**Claims** (for the Contracting States : BE, CH, DE, FR, GB, IT, LI, NL, SE)

1. A 7-phenyl-7-phenoxymethyl-hexahydro-1,4-oxazepine of the formular I

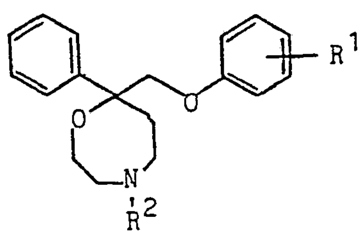

(I)

where

$R^1$ is hydrogen, chlorine or methoxy, and

$R^2$ is hydrogen or methyl,
and its salts physiologically tolerated acids.

2. 7-Phenyl-7-phenoxymethyl-hexahydro-1,4-oxazepine.

3. A process for the preparation of a compound of the formula I as claimed in claim 1, wherein

a) where $R^2$ is methyl, a compound of the formula II

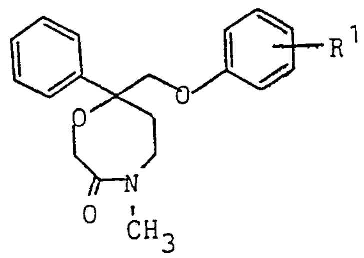

(II)

where $R^1$ has the above meanings, is reduced with a strong reducing agent, or

b) where $R^2$ is hydrogen, a compound of the formula III

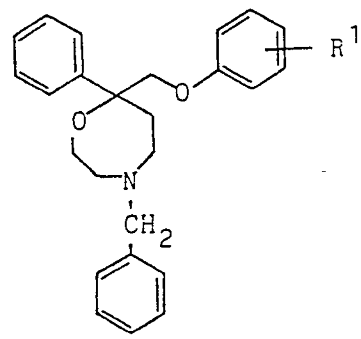

(III)

where $R^1$ has the above meanings, is reductively debenzylated, and, if desired, the resulting compound is methylated at the nitrogen atom and/or is converted into its salts with physiologically tolerated acids.

4. A drug containing a compound as claimed in claim 1.

5. A compound as claimed in claim 1 for use in the treatment of psychic disorders.

**Claims** (for the Contracting State AT)

1. A process for the preparation of a 7-phenyl-7-phenoxy-methyl-hexahydro-1,4-oxazepine of the formula I

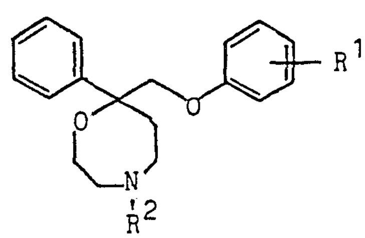

(I)

where
$R^1$ is hydrogen, chlorine or methoxy, and
$R^2$ is hydrogen or methyl,
and its salts with physiologically tolerated acids, wherein

a) where $R^2$ is methyl, a compound of the formula II

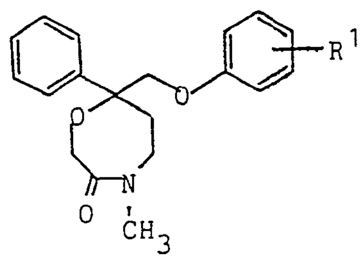

(II)

where $R^1$ has the above meanings, is reduced with a strong reducing agent, or

b) where $R^2$ is hydrogen, a compound of the formula III

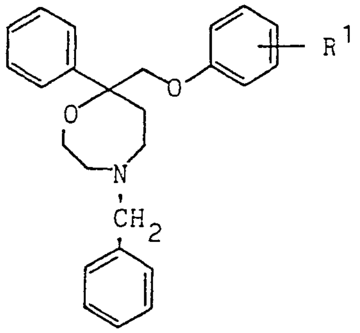

(III)

where $R^1$ has the above meanings, is reductively debenzylated, and, if desired, the resulting compound is methylated at the nitrogen atom and/or is converted into its salts with physiologically tolerated acids.

2. A process as claimed in claim 1, wherein 7-phenyl-7-phenoxy-methyl-hexahydro-1,4-oxazepine is produced.

**Revendications** (pour les Etats contractants : BE, CH, DE, FR, GB, IT, LI, NL, SE)

1. Phényl-7 phénoxyméthyl-7 hexahydro-oxazépines-1,4 de la formule I

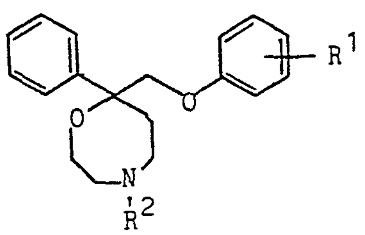

(I)

dans laquelle

$R^1$ désigne un atome d'hydrogène ou de chlore ou un groupe méthoxy et

$R^2$ un atome d'hydrogène ou un radical méthyle,

ainsi que leurs sels d'acides physiologiquement compatibles.

2. La phényl-7 phénoxyméthyl-7 hexahydro-oxazépine-1,4.

3. Procédé de préparation de composés de la formule I selon la revendication 1, caractérisé en ce que :

a) si $R^2$ est un radical méthyle, on réduit un composé de la formule II

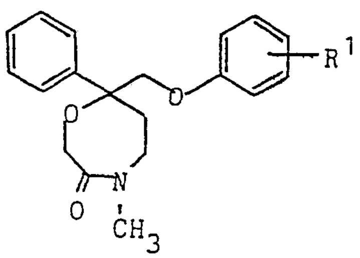

(II)

dans laquelle $R^1$ possède la signification indiquée, à l'aide d'un agent de réduction fort ;

b) si $R^2$ est un atome d'hydrogène, on soumet un composé de la formule III

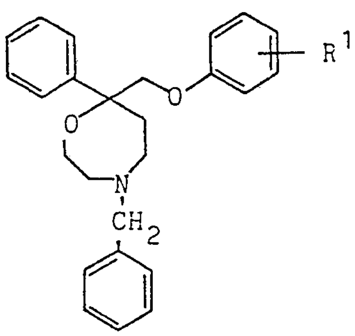

(III)

dans laquelle $R^1$ possède la signification indiquée, à une débenzylation réductrice, les composés ainsi obtenus pouvant éventuellement être soumis à une méthylation sur l'atome d'azote ou transformés en sels d'acides physiologiquement acceptables.

4. Médicament, contenant un composé selon la revendication 1.

5. Composé selon la revendication 1, utilisé dans le traitement de maladies psychiques.

**Revendications** (pour l'Etat contractant AT)

1. Procédé de préparation de phényl-7 phénoxyméthyl-7 hexahydro-oxazépines-1,4 de la formule I

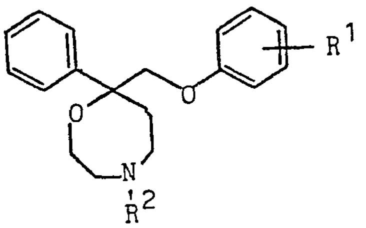

(I)

dans laquelle

$R^1$ désigne un atome d'hydrogène ou de chlore ou un groupe méthoxy et

$R^2$ un atome d'hydrogène ou un radical méthyle,

ainsi que de leurs sels d'acides physiologiquement compatibles, caractérisé en ce que :

a) si $R^2$ est un radical méthyle, on réduit un composé de la formule II

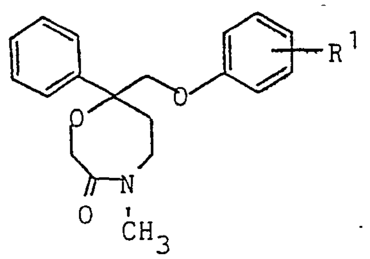

(II)

dans laquelle $R^1$ possède la signification indiquée, à l'aide d'un agent de réduction fort ;

b) si $R^2$ est un atome d'hydrogène, on soumet un composé de formule III

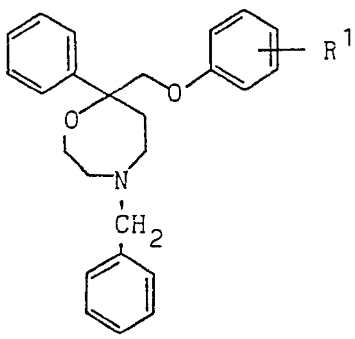

(III)

dans laquelle $R^1$ possède la signification indiquée, à une débenzylation réductrice, les composés ainsi obtenus pouvant éventuelement être soumis à une méthylation sur l'atome d'azote ou transformés en sels d'acides physiologiquement acceptables.

2. Procédé suivant la revendication 1, caractérisé en ce qu'il est appliqué à la préparation de la phényl-7 phénoxyméthyl-7 hexahydro-oxazépine-1,4.